# EUROPEAN PATENT APPLICATION

(11) **EP 2 277 453 A1**
(43) Date of publication of application: **26.01.2011**
(21) Application number: 09746672.6
(22) Date of filing: 15.05.2009
(51) Int. Cl.: A61B 10/00, A61B 6/03, G01N 21/64, G01N 21/78

(54) **DATA COLLECTION METHOD**

(30) Priority: 15.05.2008 US 53249
(71) Applicant: Osaka Prefectural Hospital Organization, Osaka 558-8558 (JP); Hamamatsu Photonics K.K., Hamamatsu-shi, Shizuoka 435-8558 (JP)
(72) Inventor: ISHIKAWA Osamu, Osaka-shi Osaka 537-8511 (JP); YAMADA Terumasa, Osaka-shi Osaka 537-8511 (JP); GOTOH Kunihito, Osaka-shi Osaka 537-8511 (JP); MIWA Mitsuharu, Hamamatsu-shi Shizuoka 435-8558 (JP); SHIKAYAMA Takahiro, Hamamatsu-shi Shizuoka 435-8558 (JP)
(74) Representative: HOFFMANN EITLE
(86) International application number: PCT/JP2009/059059
(87) International publication number: WO 2009/139466

(57) **Abstract**

Provided is a data collection method, comprising: comparing a near-infrared fluorescence intensity distribution image, obtained by emitting an indocyanine green excitation light on a target organ in a living body administered with indocyanine green, with a cancer lesion distribution image, obtained by employing x-ray, nuclear magnetic resonance or ultrasound on the target organ before administering the indocyanine green; and collecting data on areas, being detected by the near-infrared fluorescence intensity distribution image but undetected by the cancer lesion distribution image, as accessory cancer lesion area data.

## Description

### Technical Field

The present invention relates to a data collection method, more specifically, a data collection method for accessory cancer lesion areas. The present invention further relates to an accessory cancer lesion detection agent and composition thereof, and a device used for detecting accessory cancer lesions.

### Background Art

Computer tomography (CT), nuclear magnetic resonance imaging (MRI), and ultrasonic apparatus are widely used as methods of imaging cancer lesions. For example, contrast CT, wherein an iodinated contrast agent having high x-ray absorption is injected into a blood vessel (normally a peripheral vein), is generally used for testing cancer by CT (see, for example, Patent Document 1).

### Citation List

### Patent Literature

Patent Document 1: Japanese Unexamined Patent Application Publication No. 2007-533737

### Summary of Invention

### Technical Problem

However, even when the cancer lesions are identified by a method described above, or any other known imaging method, and resected, this does not lead to complete cure of the cancer in many cases.

For example, the 5-year recurrence-free survival of patients who have undergone radical resection of hepatocellular carcinoma (HCC) is as low as about 30%. One suspected reason for this low survival, especially in cases of early recurrence that occurs within 2 years post surgery is that the accessory cancer lesions that cannot be detected by conventional preoperative examination or intraoperative macroscopic observation are missed in the resection. Thus, there is a need to improve the sensitivity of detecting the accessory lesions before and during the surgery, in order to completely cure the cancer.

The object of the present invention is therefore to provide a data collection method for accessory cancer lesion areas in order to assist diagnosis.

### Solution to Problem

The present invention provides a data collection method comprising: comparing a near-infrared fluorescence intensity distribution image, obtained by emitting an indocyanine green excitation light on a target organ in a living body administered with indocyanine green (ICG), with a cancer lesion distribution image, obtained by employing x-ray, nuclear magnetic resonance or ultrasound on the target organ before administering the indocyanine green; and collecting data on areas, being detected by means of the near-infrared fluorescence intensity distribution image but undetected by means of the cancer lesion distribution image, as accessory cancer lesion area data.

According to this method, the accessory cancer lesion data (including boundary information, such as location, size, etc, of the accessory cancer lesions) can be collected from areas of the target organ that are not detected by x-ray CT, MRI, ultrasonography or macroscopic observation. In other words, the data collection method of the present invention is a method of collecting data on a human body for assisting the final diagnosis (but excluding surgical, therapeutic or diagnostic processes). The use of such data can prevent the missing of minute accessory cancer lesions by doctors, and improve the postoperative survival of the patient. In the present invention, lesions detected in the cancer lesion distribution images obtained by employing x-ray, nuclear magnetic resonance or ultrasound on the target organ before administering the indocyanine green are termed "main cancer lesions".

It is preferable to obtain the near-infrared fluorescence intensity distribution image of the target organ of the living body after intravenous injection of indocyanine green, and it is preferable to obtain the intensity distribution image of the target organ of the living body 1 to 10 days after indocyanine green administration.

Furthermore, it is preferable to obtain the near-infrared fluorescence intensity distribution image of the target organ in the living body administered with indocyanine green that is neither complexed with a high density lipoprotein nor bound to antibodies against proteins specifically present in accessory cancer lesions. However, the near-infrared fluorescence intensity distribution image can also be obtained from the target organ in the living body to which indocyanine green in the form of the above-described complex, or indocyanine green bound to the above-described antibodies, has been administered. Cancers targeted by the method include primary hepatocellular cancer and metastatic hepatocellular cancer, and it can be applied in the living body of a human or non-human mammal.

As a method of examining hepatic function, it has been the conventional practice to examine hepatic function by identifying the processing capacity of the liver through injecting indocyanine green (dye) into a vein in an arm (intravenous injection), collecting a blood sample after a given time has passed and quantifying the dye remaining in the blood. However, this is the first time that the inventors have discovered the phenomenon of indocyanine green accumulating to detectable levels, not just in the main lesions but also extending to accessory lesions, through intravenously injecting indocyanine green being neither complexed with a high density lipoprotein nor bound to antibodies against proteins that are specifically present in the cancerous tissue.

From the above, it is clear that indocyanine green functions as an accessory cancer lesion detection agent. Specifically, indocyanine green, being neither complexed with a high density lipoprotein nor bound to antibodies against proteins that are specifically present in the cancer lesions, functions as the accessory cancer lesion detection agent. Accessory lesions to be detected include those present in areas that cannot be detected by x-ray, MRI, ultrasound or macroscopic observation.

Indocyanine green does not have to be used as a simplex, but can also be used as a composition for an accessory cancer lesion detection agent comprising distilled water.

The above-described data collection method can be used for the device described below. In other words, one can use an accessory cancer lesion detector, which detects accessory cancer lesions during surgery for shrinking, destruction or resection of cancer lesions, comprising an irradiation means for emitting an indocyanine green excitation light on a target organ, suspected of having cancer, in a living body administered with the indocyanine green, and an imaging means for capturing the near-infrared fluorescence intensity distribution image from the excited indocyanine green in respect to the target organ.

In this device, it is preferable that the irradiation means and imaging means are installed in an integrated manner so that the device can be brought close to the site of the accessory lesions exposed by the surgery. This type of configuration enables image capturing, for instance, by bringing the device close to the cancer lesions after an abdomen has been surgically opened.

### Advantageous Effects of Invention

The present invention can provide a data collection method for accessory cancer lesion areas in order to assist diagnosis. This consequently enables the detection of accessory cancer lesions, and thus can improve the 5-year recurrence-free survival of patients who undergo radical cancer surgery.

### Brief Description of Drawings

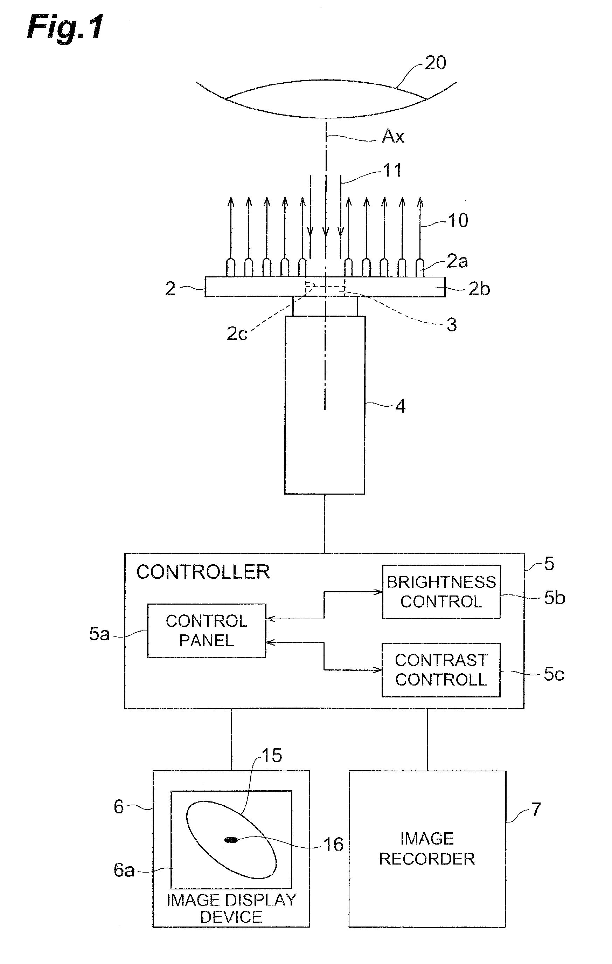

FIG. 1 is a configuration diagram of an embodiment of the accessory cancer lesion detector;

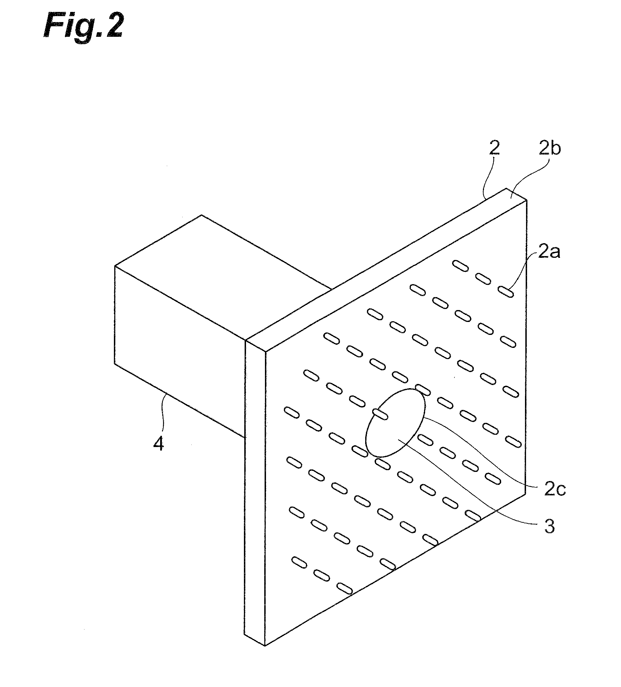

FIG. 2 is a perspective view showing the configuration of the excitation light source unit and imager used in the detector shown in FIG. 1;

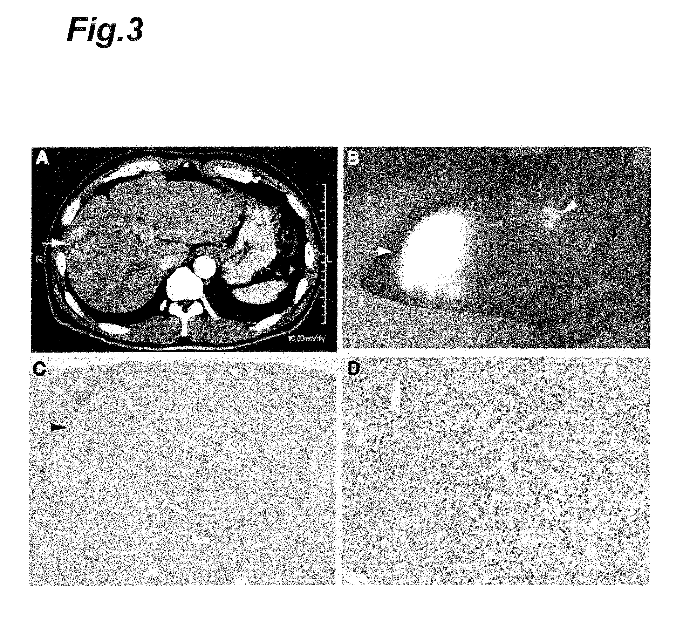

FIG. 3 shows images of cancer lesions obtained by contrast CT and the ICG fluorescence method, and micrographs of a cancer lesion; A is an image obtained by contrast CT, B is a near-infrared fluorescence intensity distribution image from ICG in the liver, C is a micrograph of a tissue section of an accessory lesion, and D is a magnified image of the micrograph of 3C;

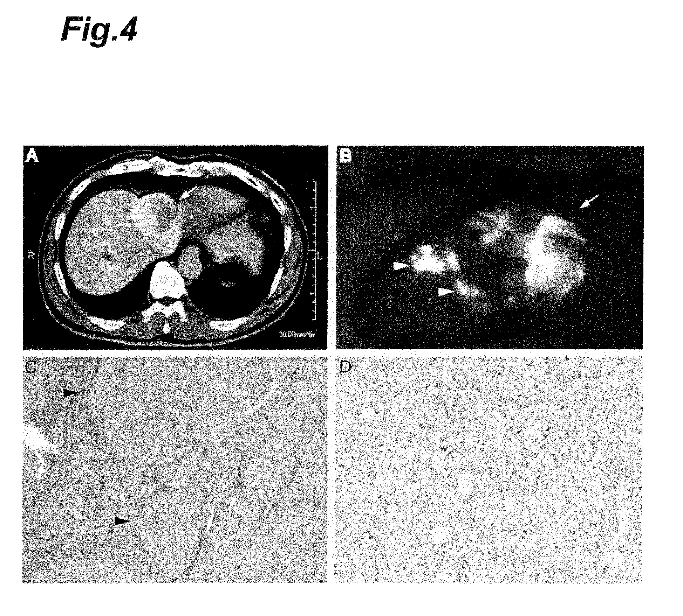

FIG 4 shows images of cancer lesions obtained by contrast CT and the ICG fluorescence method, and micrographs of a cancer lesion; A is an image obtained by contrast CT, B is a near-infrared fluorescence intensity distribution image from ICG in the liver, C is a micrograph of a tissue section of an accessory lesion, and D is a magnified image of the micrograph of C;

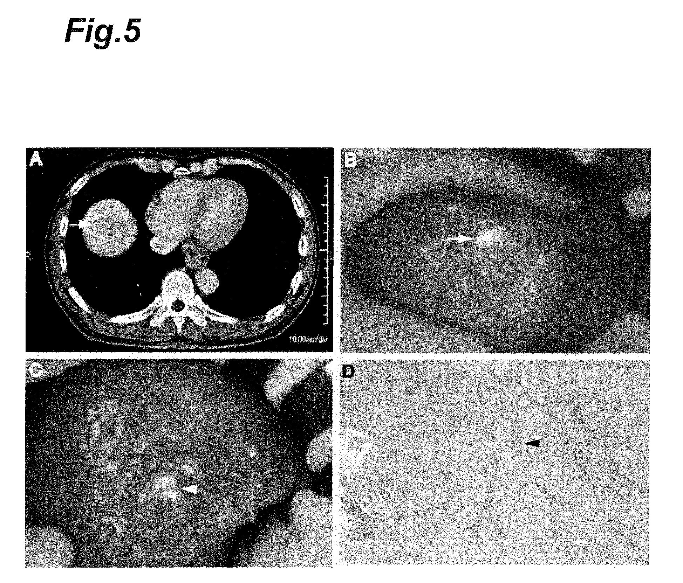

FIG. 5 shows images of cancer lesions obtained by contrast CT and the ICG fluorescence method, and micrographs of a cancer lesion; A is an image obtained by contrast CT, B and C are near-infrared fluorescence intensity distribution images from ICG in the liver, and D is a micrograph of a tissue section of an accessory lesion;

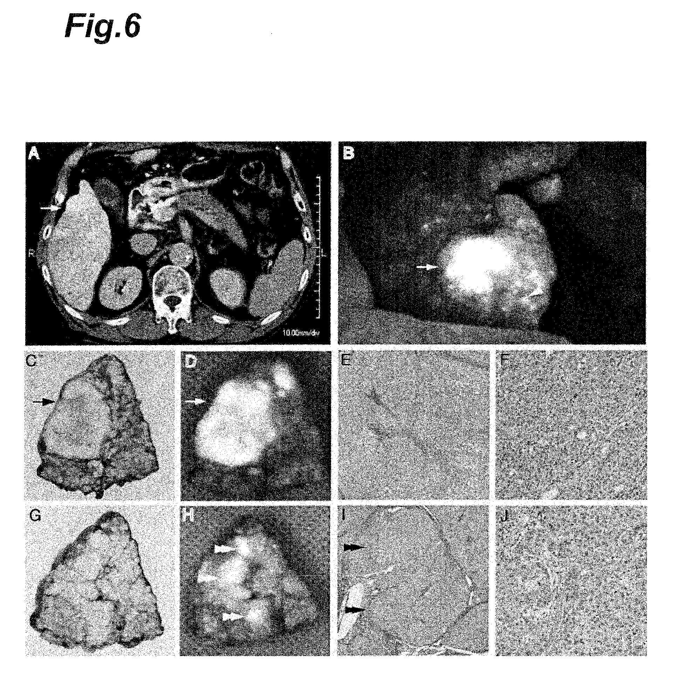

FIG. 6 shows images of cancer lesions obtained by contrast CT and the ICG fluorescence method, photographs of resected cancer lesions, and micrographs of cancer lesions; A is an image obtained by contrast CT, B is a near-infrared fluorescence intensity distribution image from ICG in the liver, C and G are photographs of formalin-fixed liver, D and H are near-infrared fluorescence intensity distribution images from ICG in the resected liver (they respectively correspond to C and G), and E, F, I, and J are micrographs of lesion tissue (F is a magnified image of E and J is a magnified image of I); and

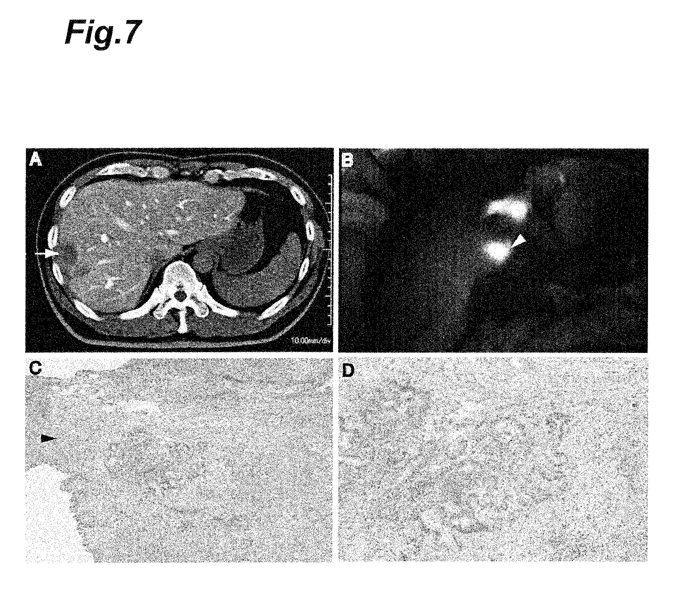

FIG. 7 shows images of cancer lesions obtained by contrast CT and the ICG fluorescence method, and micrographs of the cancer lesions; A is an image obtained by contrast CT, B is a near-infrared fluorescence intensity distribution image from ICG in the liver, C is a micrograph of a tissue section of an accessory lesion, and D is a magnified image of the micrograph of C.

### Description of Embodiments

A preferred embodiment is described below, referring to the drawings. In the description of the drawings, identical symbols are assigned to the same components, and duplication of descriptions is avoided. Furthermore, parts of the drawings are exaggerated to facilitate better understanding, and the dimensional proportions do not always match with those of the components described.

In the data collection method of the present invention, a near-infrared fluorescence intensity distribution image is obtained by emitting an indocyanine green excitation light onto a targeted organ of a living body that has been administered with indocyanine green, and an cancer lesion distribution image is obtained by employing x-ray, nuclear magnetic resonance or ultrasound on a targeted organ prior to administering indocyanine green.

The targeted organ for which a near-infrared fluorescence intensity distribution image is obtained is a target organ of a living body that has been administered with indocyanine green, preferably by intravenous injection. It is especially preferable to use indocyanine green that is neither complexed with a high density lipoprotein nor bound to antibodies against proteins specifically present in accessory cancer lesions. The indocyanine green is preferably injected intravenously with distilled water as a composition for accessory cancer lesion detection. The amount of indocyanine green in the composition for accessory cancer lesion detection can be appropriately decided according to such factors as the type of cancer and level of progression as well as the age and weight of the patient (animal). The near-infrared fluorescence intensity distribution image is preferably obtained prior to obtaining the cancer lesion distribution image by employing x-ray, nuclear magnetic resonance or ultrasound.

The indocyanine green excitation light emitted onto the target organ is preferably a near-infrared light (preferably 700-1000 nm, or more preferably 700-800 nm). Specifically, it is desirable to emit the excitation light by a light emitting diode (LED) or laser diode (LD). Furthermore, a method of providing an optical filter (e.g., a low-pass filter transmitting wavelengths of 800 nm or less, or a band-pass filter with a center wavelength of 760 nm) on a halogen lamp of approximately 150W as a light source and emitting the light therefrom by optical fiber may also be employed. The excitation light intensity and excitation time can be appropriately decided according to such factors as the amount of indocyanine green introduced and the size of the cancer lesion.

Since near-infrared fluorescence from excited indocyanine green typically has a wavelength of 800-900 nm (specifically, 825-850 nm) a near-infrared fluorescence intensity distribution can be obtained by a means capable of imaging this wavelength of light. An example of an imaging means is a solid state imaging device such as a CCD camera. When using a CCD camera, it is preferable to first remove the infrared cut-off filter.

The near-infrared fluorescence intensity distribution image may be obtained from the target organ 1 to 10 days (more preferably, 3 to 5 days) after the indocyanine green has been administered. If the imaging is done soon after the administration of the indocyanine green (less than 1 day after administration), near-infrared fluorescence is sometimes obtained from all parts of the target organ, and there is the possibility of not being able to differentiate the lesion from normal tissue. Beyond 10 days after administration, the near-infrared fluorescence sometimes becomes faint even in the lesion.

The present inventors do not wish to adhere to one particular theory, but assume that the indocyanine green gets accumulated in the neovascularized parts of the cancer lesions. New blood vessels are continuously formed one after the other in cancer lesions. It is conceivable that the indocyanine green can easily leak out from the newly formed blood vessels, and therefore, it gets accumulated around the newly formed vessels.

It is suitable to obtain the near-infrared fluorescence intensity distribution images 2-dimensionally or 3-dimensionally all over the target organ. In this manner, the location of the cancer lesions (main lesion and/or accessory lesions) that are the targets of shrinking, destruction or resection can be accurately identified.

The cancer lesion distribution image is obtained by employing x-ray, nuclear magnetic resonance or ultrasound on the target organ before the indocyanine green administration. The lesions detected by such imaging are the main cancer lesions, and the detectable size of such lesions is about 5 mm in the long axis direction.

A cancer lesion distribution image obtained by x-ray, i.e., an x-ray image (CT), can be captured using, for instance, the Aquilion 16 (trademark) medical x-ray CT system (manufactured by Toshiba Medical Systems Corporation) or similar equipment. The nonlimiting exemplified conditions for capturing x-ray image (CT) of a liver are a tube voltage of 120 kV, a tube current of 400 mA, a rotation time of 0.5 sec/rotation, a pitch of 1.5, and a slice thickness of 1 mm. An example of an iodinated contrast agent is Iopamiron (registered trademark; Bayer HealthCare), which has iopamidol (generic name) as the active ingredient. For instance, 370 mg (95 mL) of Iopamiron may be injected at the rate of 4 mL/sec. A scan delay time of 20 sec may be used in the case of an early arterial phase (screening), about 30 sec for a late arterial phase (detailed examination), and about 80 sec for a portal venous phase (detailed examination). The dose of the x-ray is to be set according to the size of a patient.

The cancer lesion distribution image obtained by nuclear magnetic resonance, i.e., a nuclear magnetic resonance image (MRI), can be captured by using, for instance, the MAGNETOM Symphony 1.5T (manufactured by Siemens AG) MRI system or similar equipment. The procedure for capturing the image is not specifically restricted and is exemplified by the following method. Firstly, before injection of the contrast agent, slice images along the three axis directions, i.e., coronal, sagittal, and transverse, are captured under a static condition. Next, the contrast agent is injected, and dynamic imaging is carried out. More specifically, after deciding the imaging site, a bolus of the contrast agent is injected intravenously, and the temporal changes in the three axis directions are captured at intervals of about 10 sec. In some cases the imaging may be done along one axis direction only. Finally, after sufficient time has lapsed since the injection of the contrast agent, slice images along the three axis directions are captured as static contrast-enhanced images. Among these nuclear magnetic resonance images, the dynamic images are particularly effective for assessing the quality of cancer lesions.

The cancer lesion distribution image obtained by ultrasound, i.e., an ultrasound image, can be captured, for instance, using the digital diagnostic ultrasound system EUB-8500 (manufactured by Hitachi Medical Corporation) or similar equipment. The imaging frame rate and power level can be set by a person skilled in the art, based on the location, size, etc of the target organ.

Data on the accessory cancer lesion area is then collected from the two types of images obtained by the methods described above. In other words, data on the area detected by the near-infrared fluorescence intensity distribution image, but not detected in the cancer lesion distribution image is collected as the data on the accessory cancer lesion area.

The accessory cancer lesion area data can be identified by superimposing the near-infrared fluorescence intensity distribution image and the cancer lesion distribution image. Such superimposition can be done manually or by superimposing digital images. This identification of the accessory cancer lesion area data is carried out without an intervention of a doctor. The two images can be compared, for instance, by identifying, from the cancer lesion distribution image, the organ wherein the main cancer lesion is present, and then determining the location of the identified organ in the near-infrared fluorescence intensity distribution image. In other words, the area detected in the near-infrared fluorescence intensity distribution image but not in the cancer lesion distribution image can be discovered. The data collection method of the present invention is particularly useful when the accessory cancer lesion is present in an organ that cannot be detected in the cancer lesion distribution image. However, the method can also be used for detecting lesions (accessory lesions) in parts that cannot be detected in the cancer lesion distribution image even though the organ itself can be detected in that image. By using the data collection method of the present invention, cancer lesions (accessory lesions) with a long axis length of less than 5 mm can be detected.

The cancer targeted by the present invention is preferably a solid cancer (primary cancer) such as gastric cancer, esophageal cancer, colon cancer, liver cancer, etc., but it can be a metastatic cancer that has metastasized from a cancer of some other organ. Among the solid cancers, the method of the present invention is suitable for liver cancer, hepatocellular carcinoma in particular, because patients of such cancers have low postoperative survival. Furthermore, the subject (living body) to be tested can be a human or non-human mammal.

As described above, the indocyanine green functions as the accessory cancer lesion detection agent. In other words, the indocyanine green functions as a detection agent that detects accessory lesions present in areas where no lesions are detected by x-ray imaging, MRI, ultrasonic apparatus, or macroscopic observation. Indocyanine green that is neither complexed with a high density lipoprotein, nor bound to antibodies against proteins specifically present in the accessory lesions, is suitable.

The accessory cancer lesion detection agent is effective for liver cancer, especially hepatocellular carcinoma. It is preferable to use for conducting detection 1 to 10 days after an administration to a living body (human or non-human mammal).

FIG. 1 is a configuration diagram of an embodiment of an accessory cancer lesion detector, which can be used in the data collection method of the present invention. FIG. 2 is a perspective view showing a configuration of an excitation light source unit and an imager used in the accessory cancer lesion detector shown in FIG. 1.

The accessory cancer lesion detector of the embodiment shown in FIG. 1 irradiates an excitation light 10 of a certain wavelength onto a target organ 20, and observes the image created by fluorescence (fluorescence image 11) emitted by the target organ 20, to detect an accessory cancer lesion. In the detection of accessory cancer lesion using this detector, indocyanine green is injected beforehand at a point near the lesion in the target organ 20 or vein. Then, near-infrared fluorescence coming from the indocyanine green accumulated in the accessory cancer lesion is observed to detect the accessory cancer lesion.

The accessory cancer lesion detector shown in FIG. 1 comprises an excitation light source unit 2 (irradiation means), an optical filter 3, an imager 4 (imaging means), a controller 5, and an image display device 6. The excitation light source unit 2 has a plurality of excitation light sources 2a, and a support plate 2b on one side whereof the excitation light sources 2a are installed. Each of the excitation light sources 2a comprises a light source that radiates light of the same wavelength as the excitation light, and is used to irradiate the target organ 20 with the excitation light 10. As shown in FIG. 2, the excitation light sources 2a are arranged two-dimensionally with symmetry about the central axis Ax of the excitation light source unit 2, which will become the optical axis of the detector.

As mentioned earlier, it is preferable to use semiconductor lasers (LD) or light emitting diodes (LED) as the excitation light sources 2a. The wavelength of the excitation light 10 supplied by the excitation light sources 2a is suitably selected (760 nm for instance) from the near-infrared wavelength band, as the absorption band of indocyanine green is in that range.

An opening 2c is provided on the support plate 2b at its central location, which includes the central axis Ax. The opening 2c is provided to enable the fluorescence image 11, which comes from the target organ 20 towards the front of the excitation light source unit 2, to pass to the rear. The plurality of excitation light sources 2a described above is aligned 2-dimensionally to surround the opening 2c. In such a configuration, it is suitable to set the optical axes of those excitation light sources 2a located close to the opening 2c inclined towards the central axis Ax to prevent the intensity distribution of the excitation light 10 irradiated onto the target organ 20 from becoming weak at the center because of the effect of the opening 2c.

The optical filter 3 is installed in the opening 2c of the support plate 2b. Of the light coming from the target organ 20, which is the object of observation, this optical filter 3 allows the passage of light of the wavelength band of the fluorescence image 11 emitted by the accessory cancer lesions. A filter having transmission characteristics that cut off light of wavelengths other than that of the fluorescence image 11, which includes the excitation light 10 reflected back by the target organ 20, is preferably used as the optical filter 3.

The imager 4 is installed at the rear of the excitation light source unit 2. In this embodiment, the imager 4 is integrated with the excitation light source unit 2, with a common optical axis Ax. The fluorescence image 11 emitted by the fluorescent dye in an accessory cancer lesion, which is excited by the excitation light 10 irradiated by the excitation light sources 2a, passes through the opening 2c and the optical filter 3 of the support plate 2b, and arrives at the imager 4. The imager 4 captures the incoming fluorescence image 11 and outputs, as image data, the observed image thus obtained.

A CCD camera that can capture 2-dimensional images is used, for instance, as the imager 4. The imager is preferably capable of capturing, with high sensitivity, the light of the wavelength band of the fluorescence image 11 (the near-infrared wavelength band, as usually the target is a fluorescence image of about 800 nm). A power source for the excitation light sources and a power source for the imager are connected respectively as required with the plurality of excitation light sources 2a and the imager 4. The power sources, etc are not shown in FIG. 1. These devices can also be battery-driven.

The controller 5 is provided for the observed image output from the imager 4. The controller 5 is a means of manually or automatically controlling the image data of the observed image output by the imager 4. The controller 5 in this embodiment has a brightness control 5b and a contrast control 5c, which respectively control the brightness and contrast of the observed image output by the imager 4. The control settings of the observed image, in the controls 5b and 5c, are input from a control panel 5a. The control panel 5a sets the control settings of the observed image automatically or through inputs from the viewer. If the control settings are fixed, there is no need to provide the control panel 5a. The transmission of image data from the imager 4 to the controller 5 can be through a cable, or wireless.

The image display device 6 and an image recorder 7 are connected to the controller 5. The image display device 6 displays, on its display section 6a, the observed image 15 that has been controlled by the controller 5, as the image to be used for detecting accessory cancer lesions. Examples of image display devices 6 include a CRT monitor and a liquid crystal display, attached to a CCD camera used as the imager 4. The image recorder 7 is a means of recording the observed image data controlled by the controller 5. Examples of devices that can be used as the image recorder 7 include a videotape recorder that records the observed image on a recording medium such as videotape.

The following is an explanation of the data collection method using the accessory cancer lesion detector illustrated in FIG. 1. First, the fluorescent dye indocyanine green is injected intravenously. After the lapse of a certain time (typically 1 to 10 days from the intravenous injection), when the excitation light 10 of a certain wavelength (760 nm for instance) is irradiated on the target organ 20 from the excitation light source unit 2, the fluorescence image 11 in the near-infrared wavelength band is emitted by the accessory cancer lesion because of the indocyanine green. Here, the optical filter 3 allows the passage of the fluorescence image 11 while cutting off the reflected light coming from the target organ 20 that is being irradiated by the excitation light 10.

The fluorescence image 11 that passes through the optical filter 3 is captured by the CCD camera used as the imager 4, and the data of the observed image is output from the CCD camera to the controller 5. The controller 5 controls the brightness and contrast of the observed image coming from the imager 4. As a result, the observed image 15 (which includes the image of the main cancer lesion) containing the accessory cancer lesion image 16 is generated. When such an image is displayed on the display section 6a of the image display device 6, a near-infrared florescence intensity distribution image is obtained. Furthermore, if necessary, the observed image 15 is recorded on a recording medium in the image recorder 7. The near-infrared florescence intensity distribution image obtained in this manner is compared with the cancer lesion distribution image obtained by employing x-ray, nuclear magnetic resonance or ultrasound, thereby enabling the collection of data on accessory cancer lesion areas.

### Examples

The present invention will now be described in more specific terms, citing some examples of the present invention. However, these examples in no way restrict the scope of the invention, and various modifications can be made within the technical scope of the present invention. The method of detecting the near-infrared florescence obtained by emitting an indocyanine green excitation light is sometimes called the ICG florescence method.

In the examples 1 to 5 given below, the ICG (0.5 mg/kg) was injected intravenously a few days before the surgery. In other words, in the examples 1, 2, 3, 4 and 5, the intravenous ICG injection was administered, respectively, 4, 4, 8, 1 and 4 days before the surgery. The liver was observed with he infrared camera system PDE (trade name: Photodynamic Eye; Hamamatsu Photonics K.K.) having the configuration shown in Figures 1 and 2, used as the accessory cancer lesion detector, and hepatocellular carcinoma was detected. In examples 1 to 4, the cancer was primary liver cancer, and in Example 5 it was metastatic liver cancer.

### (Example 1)

Example 1 was a case of a male in his 50s. Preoperative contrast CT detected the presence of a single HCC, 40 mm in diameter, in the S5/8 segment of the liver. FIG. 3A is an image showing the result of contrast CT. The arrow indicates the HCC. When the patient's abdomen was cut open and intraoperative diagnosis was carried out with the PDE, data could be collected that there was a fluorescent area, 5 mm in diameter, in the S4 segment of the liver, in addition to the main tumor (main lesion). Therefore, this part (the accessory lesion) was also resected. FIG. 3B is a near-infrared fluorescence intensity distribution image (measured after the abdomen was opened but before the resection) from ICG in the liver. The arrow (arrowhead) on the right indicates the fluorescent area (accessory lesion), 5 mm in diameter, in the S4 segment of the liver. The larger fluorescent area, indicated by the arrow on the left, is the main tumor area (main lesion). Based on histological tests on the resected tissue, the main tumor was diagnosed as moderately differentiated HCC, and the accessory lesion detected in the S4 segment of the liver was diagnosed as highly differentiated HCC. FIG. 3C is a micrograph of a tissue section of the accessory lesion (fluorescent area, 5 mm in diameter, in the S4 segment of the liver), and FIG. 3D is a magnified image of the micrograph of FIG. 3C.

### (Example 2)

Example 2 was a case of a male in his 70s. Preoperative contrast CT detected the presence of a single HCC, 45 mm in diameter, in the S2/3/4 segment of the liver. FIG. 4A is an image showing the result of contrast CT. The arrow indicates the HCC. From the contrast CT image, the HCC was diagnosed to be simple nodular type. But after opening of the abdomen, and intraoperative diagnosis using the PDE, data could be collected on some fluorescent areas (accessory lesions), 2 to 3 mm in diameter, that were scattered around the main tumor (main lesion). Therefore, the area to be resected was made to include these, and the resection was carried out. FIG. 4B is a near-infrared fluorescence intensity distribution image (measured after the abdomen was opened but before the resection) from ICG in the liver. The fluorescent area indicated by the arrow on the right is the main tumor area (main lesion). The two arrows (arrowheads) on the left indicate fluorescent areas (accessory lesions), 2 to 3 in mm diameter, seen around the main tumor. Based on histological tests on the resected tissue, the main tumor was diagnosed to be a moderately differentiated HCC, and the scattered nodules (accessory lesions) were diagnosed to be satellite nodules of simple nodular HCC with extranodular growth. FIG. 4C is a micrograph of a tissue section of an accessory lesion (fluorescent area, 2 to 3 mm in diameter seen around the main tumor), and FIG. 4D is a magnified image of the micrograph of FIG. 4C.

### (Example 3)

Example 3 was a case of a male in his 60s. Preoperative contrast CT detected the presence of a single HCC, 25 mm in diameter, in the S8 segment of the liver. FIG. 5A is an image showing the result of contrast CT. The arrow indicates the HCC. When the abdomen was opened, the surface of the liver was found to be irregular. Therefore, the identification of the main tumor (main lesion) by intraoperative ultrasonography diagnosis was difficult. However, intraoperative diagnosis using the PDE enabled data to be collected on the HCC areas (main and accessory lesions) as fluorescent areas, thus making it easy to decide the area to be resected. FIG. 5B and FIG. 5C are near-infrared fluorescence intensity distribution images (measured after the abdomen was opened but before the resection) from ICG in the liver. The arrow in FIG. 5B indicates the main tumor (main lesion), and the arrow (arrowhead) in FIG. 5C indicates a fluorescent area (accessory lesion), 5 mm in diameter, in the S5 segment of the liver. Based on these measurements, the fluorescent area (accessory lesion), 5 mm in diameter, in the S5 segment of the liver was also resected in addition to the main tumor (main lesion). Based on histological tests on the resected tissue, the main tumor (main lesion) was diagnosed to be a highly differentiated HCC, and the accessory lesion in the S5 segment was diagnosed as slightly less differentiated HCC than the main tumor. FIG. 5D is a micrograph of the accessory lesion (arrowhead).

### (Example 4)

Example 4 was a case of a male in his 70s. Preoperative contrast CT detected the presence of a single HCC, 25 mm in diameter, in the S5 segment of the liver. FIG. 6A is an image showing the result of contrast CT. The arrow indicates the HCC. When the abdomen was opened, the surface of the liver was found to be irregular, and the identification of the main tumor by intraoperative ultrasonography diagnosis was difficult. But intraoperative diagnosis by the PDE enabled data to be collected on the HCC areas (main and accessory lesions) as fluorescent areas, which made it easy to decide the area to be resected. FIG. 6B is a near-infrared fluorescence intensity distribution image (measured after the abdomen was opened but before the resection) from ICG in the liver. The fluorescent area indicated by the arrow on the left is the main tumor area (main lesion), and the two arrows (arrowheads) on the right indicate fluorescent areas (accessory lesions), 3 mm in diameter, observed around the main tumor. Based on the above measurements, more than one fluorescent area (accessory lesions), each 3 mm in diameter, were resected in addition to the main tumor (main lesion). Further, the resected liver was made into 3 mm thick slices and fixed in formalin (FIG. 6C and FIG. 6G), and the cross-sections observed with the PDE (FIG. 6D and FIG. 6H). Based on histological test of the part corresponding to FIG. 6C and FIG. 6D, the main tumor and the scattered nodules were diagnosed as highly differentiated HCC. FIG. 6E is a micrograph of a tissue section of an accessory lesion (fluorescent area, 3 mm in diameter), and FIG. 6F is a magnified image of the micrograph of FIG. 6E. The near-infrared fluorescence intensity distribution image of the part corresponding the area shown in FIG. 6G (FIG. 6H) revealed light emission from areas where no nodule was detected macroscopically (double arrowheads in FIG. 6H). Based on histological test of the areas indicated by the double arrowheads in FIG. 6H, these areas were also diagnosed as highly differentiated HCC. FIG. 61 is a micrograph of tissue section showing these parts, and FIG. 6J is a magnified image of the micrograph of FIG. 61.

### (Example 5)

Example 5 was a case of a male in his 50s. Preoperative contrast CT detected the presence of a liver tumor, 25 mm in diameter, in the S2 segment and a liver tumor, 22 mm in diameter, in the S7 segment of the liver. FIG. 7A is an image showing the result of contrast CT. The arrow indicate liver tumor. When the abdomen was opened, and the PDE was used intraoperatively, apart from the two lesions identified before the surgery, data could be collected on a fluorescent area (accessory lesion), 5 mm in diameter, that was also detected in the S4 segment of the liver, which was difficult to identify definitely by palpation or intraoperative ultrasonography. Therefore, all these areas were included in the area to be resected, and liver resection performed. FIG. 7B is a near-infrared fluorescence intensity distribution image (measured after the abdomen was opened but before the resection) from ICG in the liver. The arrow (arrowhead) indicates a fluorescent area (accessory lesion), 5 mm in diameter. Histological test of the resected tissue confirmed that it had adenocarcinoma, which was diagnosed as metastasis of colon cancer into the liver. FIG. 7C is a micrograph of a tissue section of the accessory lesion (fluorescent area, 5 mm in diameter), and FIG. 7D is a magnified image of the micrograph of FIG. 7C.

In all the cases examined, minute HCC or metastatic cancers, which could not be confirmed by conventional contrast CT, could be discovered by intraoperative diagnosis with a PDE. Such minute HCC or metastatic cancers would have been missed in conventional hepatectomy. Such errors in observation are believed to be one of the reasons for the poor 5-year recurrence-free survival.

The ICG infrared camera system described above would be useful not only for intraoperative detection of minute HCC and metastatic cancer but also for determining the liver resection line, and for modifying the surgical technique.

### Explanation of Symbols

2···excitation light source unit, 2a···excitation light source, 2b···support plate, 2c···opening, 3···optical filter, 4··· imager, 5···controller, 5a···control panel, 5b···brightness control, 5c···contrast control, 6···image display device, 6a···display section, 7···image recorder, 10···excitation light, 11···fluorescence image, 15···observed image, 16···accessory cancer lesion image, 20··· target organ.

## Claims

1. A data collection method, comprising:
comparing a near-infrared fluorescence intensity distribution image, obtained by emitting an indocyanine green excitation light on a target organ in a living body administered with indocyanine green, with a cancer lesion distribution image, obtained by employing x-ray, nuclear magnetic resonance or ultrasound on the target organ before administering the indocyanine green; and
collecting data on areas, being detected by the near-infrared fluorescence intensity distribution image but undetected by the cancer lesion distribution image, as accessory cancer lesion area data.

2. The data collection method according to claim 1, wherein the near-infrared florescence intensity distribution image is obtained on a target organ in a living body administered with the indocyanine green through intravenous injection.

3. The data collection method according to claim 1 or 2, wherein the near-infrared florescence intensity distribution image is obtained on the target organ in the living body 1 to 10 days after administering the indocyanine green.

4. The data collection method according to any one of claims 1 to 3, wherein the near-infrared florescence intensity distribution image is obtained on the target organ in the living body administered with the indocyanine green being neither complexed with a high density lipoprotein nor bound to antibodies against proteins that are specifically present in the accessory cancer lesions.

5. The data collection method according to any one of claims 1 to 4, wherein the cancer is primary liver cancer or metastatic liver cancer.

6. The data collection method according to any one of claims 1 to 5, wherein the living body is a living body of a human or non-human mammal.

7. An accessory cancer lesion detection agent comprising an indocyanine green.

8. The accessory cancer lesion detection agent according to claim 7, wherein the accessory cancer lesions are those present in areas where no cancer lesions are detected by x-ray imaging, nuclear magnetic resonance imaging, ultrasound imaging, or through macroscopic observation.

9. The accessory cancer lesion detection agent according to claim 7 or 8, wherein the indocyanine green is neither complexed with a high density lipoprotein nor bound to antibodies against proteins that are specifically present in the accessory cancer lesions.

10. The accessory cancer lesion detection agent according to any one of claims 7-9, wherein the cancer is primary liver cancer or metastatic liver cancer.

11. The accessory cancer lesion detection agent according to any one of claims 7-10, wherein said accessory cancer lesion detection agent is for use in detection 1 to 10 days after being administered to the living body.

12. The accessory cancer lesion detection agent according to claim 11, wherein the living body is a living body of a human or non-human mammal.

13. A composition for detecting accessory cancer lesions, comprising the accessory cancer lesion detection agent according to any one of claims 7-12, and distilled water.

14. An accessory cancer lesion detector for detecting accessory cancer lesions during surgery in order to shrink, destroy or resect cancer lesions, comprising:
an irradiation means for emitting an indocyanine green excitation light on a target organ, suspected of having cancer, in a living body administered with the indocyanine green; and
an imaging means for capturing the near-infrared fluorescence intensity distribution image from the excited indocyanine green in respect to the target organ.

15. The accessory cancer lesion detector according to claim 14, wherein the irradiation means and the imaging means are installed in an integrated manner so that the detector can be brought close to the site of the accessory lesions exposed by the surgery.

16. The accessory cancer lesion detector according to claim 14 or 15, wherein:
the irradiation means is a near-infrared light emitting device; and
the imaging means is a solid state imager.
